# EUROPEAN PATENT APPLICATION

(11) **EP 3 231 416 A1**
(43) Date of publication of application: **18.10.2017**
(21) Application number: 15866582.8
(22) Date of filing: 08.12.2015
(51) Int. Cl.: A61K 8/34, A61K 8/02, A61K 8/44, A61K 8/49, A61Q 5/06

(54) **AEROSOL COSMETIC**

(30) Priority: 09.12.2014 JP 2014248558
(71) Applicant: Shiseido Company Ltd., Chuo-ku Tokyo 104-0061 (JP)
(72) Inventor: MATSUDA, Takashi, TSUZUKI-KU YOKOHAMA-SHI 224-8558 (JP)
(74) Representative: Merkle, Gebhard
(86) International application number: PCT/JP2015/084414
(87) International publication number: WO 2016/093236

(57) **Abstract**

An aerosol cosmetic exhibits an improved feeling of use while having high UV blocking properties. An aerosol cosmetic is filled up as a bulk solution together with a propellant in a pressure-resistant container and includes ethyl alcohol that is a main dispersion medium, an organic ultraviolet absorber, and an ester oil. The organic ultraviolet absorber includes an organic ultraviolet absorber that is solid at room temperature. The solid organic ultraviolet absorber is dissolved in the ester oil.

## Description

### RELATED APPLICATION

This application claims the priority to Japanese Patent Application No. 2014-248558, filed on December 9, 2014, the disclosure of which is incorporated herein by reference in its entirety.

### FIELD OF THE INVENTION

The present invention relates to an aerosol cosmetic, and particularly relates to the aerosol cosmetic comprising a solid ultraviolet (UV) absorber and per se capable of preventing of precipitation of the solid ultraviolet absorber.

### BACKGROUND OF THE INVENTION

In recent years, the influence of ultraviolet rays on hair or scalp has been revealed, and thus a UV screening agent is expected to be contained not only in face or body cosmetics but also in hair cosmetics.

Hair styling properties, non-sticky feeling of use, and being-shampooed are also required for hair cosmetics while containing the UV-screening agent leads to deterioration in feeling of use and shampooing (hair washability). Specifically, examples of the UV-screening agent include UV scattering agents comprising inorganic powders or the like, and organic ultraviolet absorbers. The former has an adverse effect on shampooing, and the latter has an adverse effect on the feeling of use. In particular, UV scattering agents can block ultraviolet rays in a comparatively wide wavelength region; however, because the UV scattering agents are in the form of fine powders, dispersibility thereof directly affects the UV scattering effect, and formation of aggregates leads to a reduction in the UV scattering effect. In general, volatile hydrocarbons such as LPG (liquid petroleum gas) are used as a propellant for aerosol cosmetics, and therefore it is inevitably essential to lipophilize the UV scattering agents, in order to enhance the dispersibility in the propellants. Thus the deterioration of shampooing is unavoidable.

For absorbing ultraviolet rays in a wide wavelength range using organic ultraviolet absorbers, a plurality of ultraviolet absorbers need to be employed, and if such ultraviolet absorbers precipitate in an aerosol in the container, clogging may occur in spraying.

### CITATION LIST

### PATENT LITERATURE

PATENT LITERATURE 1: JP Patent Publication No. JP2014-201541A
PATENT LITERATURE 2: JP Patent Publication No. JP4707221B
PATENT LITERATURE 3: JP Patent Publication No. JP2003-238350A

### DISCLOSURE OF THE INVENTION

### TECHNICAL PROBLEM

The present invention has been accomplished in view of the conventional arts, and an object of the present invention is to provide an aerosol cosmetic which exhibits the improved feeling of use while having high UV blocking properties.

### SOLUTION TO THE PROBLEM

In order to solve the problem, an aerosol cosmetic according to the present invention, which is filled up as a bulk solution together with a propellant in a pressure-resistant container, comprises ethyl alcohol that is a main dispersion medium, an organic ultraviolet absorber, and an ester oil, the organic ultraviolet absorber comprising an organic ultraviolet absorber that is solid at room temperature, the solid organic ultraviolet absorber being dissolved in the ester oil.
In the aerosol cosmetic, it is preferred that the solid organic ultraviolet absorber contains bis-ethylhexyloxyphenol methoxyphenyl triazine and/or diethylamino hydroxybenzoyl hexyl benzoate.
In the aerosol cosmetic, it is preferred that the ester oil is contained in an amount of 4.5 to 15 times by mass relative to the solid organic ultraviolet absorber.
It is preferred that the aerosol cosmetic further comprises a non-lipophilic powder for hair styling.
In the aerosol cosmetic, it is preferred that the non-lipophilic powder for hair styling contains calcined sericite and/or silicic acid anhydride.
In the aerosol cosmetic, it is preferred that, in the case of using the non-lipophilic powder for hair styling, a straight-chain polyether silicone active agent is used as an active agent, and more preferably, a polyglycerin-alkyl co-modified silicone active agent is used in an amount of 1 to 10% by mass in the bulk solution.
In the aerosol cosmetic, it is preferred that the ester oil (including a liquid ester ultraviolet absorber)/the solid ultraviolet absorber ≥ 4.5 (mass ratio) is satisfied, and the ethyl alcohol/the ester oil (including a liquid ester ultraviolet absorber) ≥ 1.5 (mass ratio) is satisfied.
In the aerosol cosmetic, it is preferred that the polyglycerin-alkyl co-modified silicone active agent is selected from one or more of the group consisting of bisbutyldimethicone polyglyceryl-3, carboxydecyltrisiloxane, and PEG-10/dimethicone.

Hereinafter, the configuration of the present invention will be described.
In the present invention, the organic ultraviolet absorbers are exemplified by 2-ethylhexyl p-methoxycinnamate, octocrylene, polysilicone-15, bis-ethylhexyloxyphenol methoxyphenyl triazine, and diethylamino hydroxybenzoyl hexyl benzoate. Among such absorbers, in view of the absorbable wavelength region and the content (formulation) balance, bis-ethylhexyloxyphenol methoxyphenyl triazine and/or diethylamino hydroxybenzoyl hexyl benzoate, which are solid at room temperature, are essential in the present invention.

The content of such solid ultraviolet absorbers is preferably 2 to 5% by mass in the bulk. Such solid ultraviolet absorbers are concerned in solubility in a (cosmetic) base while having excellent UV absorption properties, and are generally dissolved and added with an ester oil.

However, if the ester oil is used in a large amount in an aerosol cosmetic, a sticky feeling is given to hair or skin to which the aerosol cosmetic is applied.

Therefore, the amount of ester oil in the bulk may be 2 to 30% by mass in the present invention.

The ester oil that is suitably used in the present invention is a polar oil having an ester bond, and examples thereof may include tetra 2-pentaerythritol ethylhexanoate, glyceryl tri-2-ethylhexanoate, and diisopropyl sebacate. Further, ethylhexyl methoxycinnamate, octocrylene, and polysilicone-15, which are employed as ultraviolet absorbers in case, are also suitably used.

When the amount of the ester oil in the bulk exceeds 30% by mass, there is concern about a deterioration in feeling of use, and in contrast, when it is less than 2% by mass, the solid organic ultraviolet absorbers may precipitate in the aerosol container in some cases.

However, when a liquid ester ultraviolet absorber is used in an amount of 15% by mass based on the bulk or more, there is a tendency that the stickiness increases and the feeling of use deteriorates.

Further, in the present invention, it is preferred that the content of ester oil is at least 4.5 times of the content of the solid ultraviolet absorbers, and that the content of ethyl alcohol is at least 1.5 times of the content of ester oil.

Within the above range, the product stability is exceptionally good.

Further, in the present invention, when bisbutyldimethicone polyglyceryl-3, carboxydecyltrisiloxane, and/or PEG-10/dimethicone are/is selected as a polyglycerin-alkyl co-modified silicone active agent, the redispersibility in the bulk state can be improved.

Further, non-lipophilic hair styling powders are used in the present invention. The hair styling powders can enhance the hair styling qualities by adhering to hairs to increase the friction between hairs to each other, and can suppress the mal-effect in the feeling of use such as stickiness, unlike hair styling oils or resins.

In the present invention, talc, calcined sericite, silicic acid anhydride, or the like can be used as the non-lipophilic hair styling powder.

For improving the feeling of use in the present invention, silicone powders (such as a crosslinked silicone-mesh silicone block copolymer and crosslinked methylpolysiloxane), crosslinked polymethylmethacrylate, or the like can be used. Although these powders, unlike the UV scattering agent, are remarkably effective in improving the feeling of use because of a large particle size, the powders have no adverse effect on shampooing.

Further, in the present invention, it is suitable to add an active agent in order to enhance the stability of the organic ultraviolet absorbers or the ester oil.

Examples of the active agents that are effective in the present invention can include polyether-based, preferably, polyglycerin-alkyl modified silicone-based active agents, and particularly preferable examples can include bisbutyldimethicone polyglyceryl-3, carboxydecyltrisiloxane, and PEG-10/dimethicone.

In the present invention, isostearic acid, oils such as light paraffin other than the ester oil, perfumes, L-arginine, Restharrow extract (*Ononis spinose* root extract), Perilla extract, Baical skullcap extract (*Scutellaria baicalensis* root extract), menthol, gingko biloba leaf extract, marine collagen, N-methyl taurine, adenosine, hop extract, Zanthoxylum extract, or the like can be further suitably included.

Further, aliphatic hydrocarbons having 3 to 5 carbon atoms such as LPG, propane, butane, pentane, and a mixture of these materials, hydrofluoroolefins such as trans-1,3,3,3-tetrafluoroprop-1-ene, hydro fluorocarbons such as 1,1-difluoroethane, and a mixed gas thereof can be mentioned as a propellant in the present invention, and the mass ratio of bulk:propellant is preferably 1:9 to 6:4.

### EFFECT OF THE INVENTION

As has been described above, the aerosol cosmetic according to the present invention allows an appropriate amount of solid organic ultraviolet absorbers to be contained while reducing the content of ester oil in an aerosol cosmetic using ethyl alcohol as a main dispersion medium.

Further, when the aerosol cosmetic needs to have hair styling properties, high hair styling properties can be given while the stickiness is suppressed by containing non-lipophilic powders such as calcined sericite or silicic acid anhydride.

### MODE FOR CARRYING OUT THE INVENTION

Hereinafter, the method for producing aerosol cosmetics of Experimental Examples and the method for evaluating each sample will be described.

Hereinafter, the present invention will be described further in detail by way of specific examples, but the present invention is not limited to the following examples. Further, the content in the following examples indicates "% by mass" unless otherwise specified.

### Production example of Experimental Examples:

An active agent and ultraviolet absorbers are added to oils, and then an alcohol is added thereto. Further, usability improving powders are added thereto, and dispersion is performed using a homomixer to make a bulk (base solution). Thereafter, the bulk (base solution) and a propellant are filled up in an aerosol can (container) at a specific ratio.

### Evaluation method of force for hair styling:

[Force for Hair styling] A sensory evaluation was performed by 10 panelists. Specifically, 3g of the sample solution was applied to hair, and a force (effect) for hair styling was determined in accordance with the following criteria.

### (Evaluation)

A+: At least 9 panelists agree the force for hair styling is high.
A: 7-8 panelists agree the force for hair styling is high.
B: 5-6 panelists agree the force for hair styling is high.
C: No more than 4 panelists agree the force for hair styling is high.

### Method for evaluating stickiness in hair styling:

[Stickiness] After application to hair, a sensory evaluation of the texture was performed by 10 panelists, and determination was made in accordance with the following criteria.

### (Evaluation)

A+: At least 9 panelists agree the texture is not sticky.
A: 7-8 panelists agree the texture is not sticky.
B: 5-6 panelists agree the texture is not sticky.
C: No more than 4 panelists agree the texture is not sticky.

### Fluffy styling effect (volumized hair)

[Fluffy (volumized hair) feeling] After application to hair, a sensory evaluation of the texture was performed by 10 panelists, and determination was made in accordance with the following criteria.

### (Evaluation)

A+: At least 9 panelists agree the hair is volumized to be fluffy.
A: 7-8 panelists agree the hair is volumized to be fluffy.
B: 5-6 panelists agree the hair is volumized to be fluffy.
C: No more than 4 panelists agree the hair is volumized to be fluffy.

Powder redispersibility in the bulk state
A: The formulation was redispersed by shaking 1 to 5 times.
B: The formulation was redispersed by shaking 6 to 20 times.
C: The formulation was redispersed by shaking 21 to 50 times.

Powder redispersibility in the aerosol state
A: The formulation was redispersed by shaking 1 to 5 times.
B: The formulation was redispersed by shaking 6 to 20 times.
C: The formulation was redispersed by shaking 21 to 50 times.

### Method for evaluating stability of the bulk:

The precipitation of poorly soluble solid ultraviolet absorbers was checked after the container was allowed to stand at room temperature for 2 weeks after emulsification.
A: No poorly soluble solid absorbers precipitated.
B: Poorly soluble solid absorbers precipitated.

### Method for evaluating stability of product:

Aerosol products were allowed to stand still at room temperature, 0°C, and -5°C, and precipitation of poorly soluble solid ultraviolet absorbers was checked after standing for 2 weeks, 2 months, and 1 year.
A: No poorly soluble solid absorbers precipitated.
B: Poorly soluble solid absorbers precipitated.

Hereinafter, embodiments of the present invention will be described.

First, the inventors made an attempt to impart UV blocking properties to hair styling aerosol cosmetics by the method set forth above. Specifically, aerosol cosmetics have advantages such as the ease of use for consumers, the uniform application to skin, hair, or the like, and further the capability of suppressing oxidation of the contents, whereas they become unusable when the components precipitate to cause clogging. Therefore, when preparing aerosol cosmetics, it is typical to first form a "bulk" (base solution), in which cosmetic components are dissolved and dispersed in a dispersion medium such as water. A stable composition is prepared in the form of a bulk, and the composition is added to a propellant such as LPG, so that the precipitation of the components in the container is suppressed.

In particular, solid ultraviolet absorbers tend to precipitate during product storage, but the precipitation of the solid ultraviolet absorbers in the container can be suppressed by dissolving these solid ultraviolet absorbers in ester oil to prepare an emulsified bulk and further dispersing the bulk in a propellant.

**[Table 1]**

| Experimental Example | 1-1 | 1-2 | 1-3 | 1-4 | 1-5 |
|---|---|---|---|---|---|
| Deionized water | 15 | 30 | 0 | 0 | 0 |
| Ethyl alcohol | 5 | 6 | 58 | 66 | 69 |
| Active agent | | | | | |
| Lauryl PEG-9 polydimethylsiloxyethyl dimethicone | 5 | 5 | 5 | 5 | 5 |
| Oils | | | | | |
| Isododecane | 20 | 20 | 0 | 0 | 0 |
| Decamethylcyclopentasiloxane | 5 | 0 | 0 | 0 | 0 |
| Pentaerythritol tetra 2-ethylhexanoate | 5 | 6 | 5 | 0 | 0 |
| Diisopropyl sebacate | 10 | 10 | 10 | 0 | 0 |
| UV absorbers | | | | | |
| 2-Ethylhexyl p-methoxycinnamate | 10 | 10 | 10 | 10 | 16 |
| Diethylamino hydroxybenzoyl hexyl benzoate | 3 | 3 | 3 | 10 | 1 |
| UV scattering agent | | | | | |
| Methylpolysiloxane/Methylhydrogenpolysiloxane coated low temperature calcined zinc oxide | 10 | 0 | 0 | 0 | 0 |
| Usability improving powder | | | | | |
| (Vinyl dimethicone/methicone silsesquioxane) crosspolymer | 7 | 5 | 5 | 5 | 5 |
| Silicic acid anhydride | 0 | 1 | 0 | 0 | 0 |
| Others | | | | | |
| Perfume | q.s. | q.s. | q.s. | q.s. | q.s. |
| Concentrated glycerin | 3 | 3 | 3 | 3 | 3 |
| | | | | | |
| Bulk:propellant (LPG) | 5:5 | 5:5 | 5:5 | 5:5 | 5:5 |
| | | | | | |
| Ester oil (including liquid UV absorber)/solid absorber | 8.3 | 8.7 | 8.3 | 1.0 | 16.0 |
| Ethyl alcohol/ester oil (including liquid UV absorber) | 0.20 | 0.23 | 2.32 | 6.60 | 4.31 |
| Evaluation | | | | | |
| Shampooing | B | A | A | A | A |
| Stickiness in hair styling | B | B | B | C | C |
| Bulk stability (2 weeks later) | A | A | B | B | A |
| Product stability (2 months later) | A | A | A | B | A |
| Product stability (1 year later) | A | A | A | B | A |

However, as shown in Table 1, although the hair styling aerosol cosmetic using an emulsified bulk has no problems in the product stability, the shampooing and the stickiness are not satisfactory (Experimental Example 1-1). Note that, the shampooing quality is improved by removing the UV scattering agent (Experimental Example 1-2). The powder surfaces need to be hydrophobized because the UV scattering agent comprises fine particle powders and an aggregation of the fine particle powders in the container causes a significant reduction in the UV scattering effect. If thus the hydrophobized powders adhere to hair or the like, it is difficult to wash and remove the powders immediately using a common shampoo. However, it has been difficult to improve the stickiness in a hair styling when the emulsified bulk is employed.

On the other hand, in the case of using ethyl alcohol as a dispersion medium and adding a solid ultraviolet absorber that has been dissolved in an ester oil, the bulk forms a homogeneous system instead of an emulsified system (Experimental Example 1-3). Then, an improvement in stickiness in hair styling is also appreciated.

However, the bulk forming the homogeneous system had precipitation that seemed likely the ultraviolet absorbers after storage for 2 weeks, 2 months, and 1 year, and consequently such bulk is suggested to be unsuitable as a product.

The reason is probably that in the emulsified systems of Experimental Examples 1-1 and 1-2, the ester oil is dispersed in water and the ultraviolet absorbers are dissolved in the ester oil in a high concentration, but the ester oil is dissolved in ethyl alcohol and diluted, in Experimental Example 1-3, so that the dissolved state of the solid ultraviolet absorbers in the diluted ester oil/ethyl alcohol dispersion medium cannot be maintained.

On the other hand, the aerosol cosmetics tested for the hair styling qualities and shampooing by the inventors has not provided, surprisingly, the recognizable precipitation of the ultraviolet absorbers after they were allowed to stand for 2 months.

As a result of studies on this point by the inventors, it turned out that a product system in which at least a propellant was also present showed good stability by dissolving the solid ultraviolet absorbers in the ester oil, and further adjusting the content of ethyl alcohol to at least 1.5 times as much as the content of the ester oil, as described below.

It should be noted that, when the amount of the ester ultraviolet absorbers in the bulk is 15% by mass or more, the stickiness occurs (Experimental Example 1-5).

Therefore, the inventors studied powders which impart the hair styling properties under the conditions of a content of the ester oil of 17% by mass (27% by mass including the ester ultraviolet absorbers), the ester oil/the solid ultraviolet absorbers = 9, the ethyl alcohol/the ester oil ≥ 1.5, and adding bis-ethylhexyloxyphenol methoxyphenyl triazine and diethylamino hydroxybenzoyl hexyl benzoate, which were organic ultraviolet absorbers and solid at room temperature and particularly have a precipitation problem, to the ethanol dispersion medium.

Table 2 shows the results.

**[Table 2]**

| Experimental Example | 2-1 | 2-2 | 2-3 | 2-4 | 2-5 | 2-6 | 2-7 |
|---|---|---|---|---|---|---|---|
| Ethyl alcohol | 57 | 47 | 47 | 47 | 52 | 55 | 45 |
| Active agent | | | | | | | |
| Lauryl PEG-9 polydimethylsiloxyethyl dimethicone | 5 | 5 | 5 | 5 | 5 | 5 | 5 |
| Oils | | | | | | | |
| Glyceryl tri-2-ethylhexanoate | 7 | 7 | 7 | 7 | 7 | 7 | 7 |
| Diisopropyl sebacate | 10 | 10 | 10 | 10 | 10 | 10 | 10 |
| UV absorbers | | | | | | | |
| 2-Ethylhexyl p-methoxycinnamate | 10 | 10 | 10 | 10 | 10 | 10 | 10 |
| Bis-ethylhexyloxyphenol methoxyphenyl triazine | 3 | 3 | 3 | 3 | 3 | 3 | 3 |
| Usability improving powder | | | | | | | |
| (Vinyl dimethicone/methicone silsesquioxane) crosspolymer | 5 | 5 | 5 | 5 | 5 | 5 | 5 |
| Hair stiling powder | | | | | | | |
| (Dimethicone/vinyl dimethicone) crosspolymer | 0 | 10 | 0 | 0 | 0 | 0 | 0 |
| Calcined sericite | 0 | 0 | 10 | 0 | 0 | 0 | 10 |
| Methyl methacrylate crosspolymer | 0 | 0 | 0 | 10 | 5 | 2 | 2 |
| Others | | | | | | | |
| Concentrated glycerin | 3 | 3 | 3 | 3 | 3 | 3 | 3 |
| | | | | | | | |
| Bulk:propellant (LPG) | 5:5 | 5:5 | 5:5 | 5:5 | 5:5 | 5:5 | 5:5 |
| | | | | | | | |
| Ester oil (includine liquid UV absorber)/solid absorber | 9 | 9 | 9 | 9 | 9 | 9 | 9 |
| Ethyl alcohol/ester oil (including liquid UV absorber) | 2.11 | 1.74 | 1.74 | 1.74 | 1.93 | 2.04 | 1.67 |
| | | | | | | | |
| Evaluation | | | | | | | |
| Shampooing | A | A | A | A | A | A | A |
| Force for hair styling | B | B | A+ | A | A | A | A+ |
| Stickiness in hair styling | A | A | A | A+ | A | A | A+ |
| Bulk stability (2 weeks later) | B | B | B | B | B | B | B |
| Product stability (2 months later) | A | A | A | A | A | A | A |
| Product stability (1 year later) | A | A | A | A | A | A | A |

As is obvious from Table 2, precipitation in the bulk stored for 2 weeks occurred in all Experimental Examples containing the solid organic ultraviolet absorbers. However, in the LPG/ethanol system, precipitation did not occur in any case.

Further, according to the results in Table 2, the system using calcined sericite as a hair styling powder (Experimental Example 2-3) showed excellent hair styling properties, and the stickiness in hair styling was significantly improved in the system using crosslinked polymethylmethacrylate powder (Experimental Examples 2-4 to 2-6).

Further, in Experimental Example 2-7 combining the two, both the hair styling-force and the less-stickiness in hair styling were satisfactory.

Meanwhile, though not shown in Table 2, the hair styling-force is significantly reduced when the surface of the hair styling powder is lipophilized for enhancing the dispersibility of the hair styling powders in aerosol cosmetics. The reason is probably that, whereas the hair styling powders are comparatively flat and the surface thereof is rough so that the force for hair styling is exerted, the surface thereof is smoothened by the lipophilization process.

Further, hydrophilic powders such as calcined sericite having low affinity to LPG tend to precipitate and aggregate at the bottoms of containers, and further even when they are redispersed by shaking, sedimentation tends to occur earlier.

Therefore, the inventors have studied various dispersants in order to improve the aggregation resistance and redispersibility of hydrophilic powders. Table 3 shows the results.

**[Table 3]**

| Experimental Example | 3-1 | 3-2 | 3-3 | 3-4 | 3-5 | 3-6 |
|---|---|---|---|---|---|---|
| Ethyl alcohol | 40 | 40 | 40 | 40 | 40 | 40 |
| Active agent | | | | | | |
| Lauryl PEG-9 polydimethylsiloxyethyl dimethicone | 10 | 0 | 0 | 0 | 0 | 0 |
| Bisbutvldimethicone polyglyceryl-3 | 0 | 10 | 0 | 0 | 0 | 0 |
| Carboxydecyl trisiloxane | 0 | 0 | 10 | 0 | 0 | 0 |
| Cetyl PEG/PPG-10/1 dimethicone | 0 | 0 | 0 | 10 | 0 | 0 |
| PEG-10 dimethicone/polyoxyethylene-methyl polysiloxane copolymer | 0 | 0 | 0 | 0 | 10 | 0 |
| PEG-10/dimethicone | 0 | 0 | 0 | 0 | 0 | 10 |
| Oils | | | | | | |
| Glvcervl tri-2-ethylhexanoate | 3 | 3 | 3 | 3 | 3 | 3 |
| Diisopropyl sebacate | 7 | 7 | 7 | 7 | 7 | 7 |
| UV absorbers | | | | | | |
| 2-Ethylhexyl p-methoxycinnamate | 10 | 10 | 10 | 10 | 10 | 10 |
| Octocrylene | 5 | 5 | 5 | 5 | 5 | 5 |
| Diethylamino hydroxybenzoyl hexyl benzoate | 3 | 3 | 3 | 3 | 3 | 3 |
| Usability improving powder | | | | | | |
| (Vinyl dimethicone/methicone silsesquioxane) crosspolymer | 7 | 7 | 7 | 7 | 7 | 7 |
| Hair styling powder | | | | | | |
| Calcined sericite | 10 | 10 | 10 | 10 | 10 | 10 |
| Methyl methacrylate crosspolymer | 2 | 2 | 2 | 2 | 2 | 2 |
| Others | | | | | | |
| Concentrated glycerin | 3 | 3 | 3 | 3 | 3 | 3 |
| | | | | | | |
| Bulk:propellant (LPG) | 5:5 | 5:5 | 5:5 | 5:5 | 5:5 | 5:5 |
| | | | | | | |
| Ester oil (including liquid UV absorber)/solid absorber | 8.3 | 8.3 | 8.3 | 8.3 | 8.3 | 8.3 |
| Ethyl alcohol/ester oil (including liquid UV absorber) | 1.6 | 1.6 | 1.6 | 1.6 | 1.6 | 1.6 |
| Evaluation | | | | | | |
| Redispersibility in bulk state | C | A | A | C | B | A |

From the results shown in Table 3, bisbutyldimethicone polyglyceryl-3 (Experimental Example 3-2), carboxydecyl trisiloxane (Experimental Example 3-3), and PEG-10/dimethicone (Experimental Example 3-6) each have excellent effect in improving the redispersibility.

Consequently, it is understandable that polyether-based, particularly, polyglycerin-alkyl modified silicone-based active agents are suitably used as active agents in the present invention. Studies on powders were also made in consideration of the redispersibility. Table 4 shows the results.

**[Table 4]**

| Experimental Example | 4-1 | 4-2 | 4-3 |
|---|---|---|---|
| Ethyl alcohol | 55 | 60 | 55 |
| Active agent | | | |
| PEG-10/dimethicone | 3 | 3 | 3 |
| Oils | | | |
| Pentaerythritol tetra 2-ethylhexanoate | 5 | 5 | 5 |
| Diisopropyl sebacate | 7 | 7 | 7 |
| UV absorbers | | | |
| 2-Ethylhexyl p-methoxycinnamate | 10 | 10 | 10 |
| Octocrylene | 5 | 5 | 5 |
| Bis-ethylhexvloxvphenol methoxyphenyl triazine | 3 | 3 | 3 |
| Usability improving powder | | | |
| Crosslinked silicone-mesh silicone block copolymer powder | 5 | 5 | 5 |
| Hair styling powder | | | |
| Talc | 1 | 1 | 1 |
| Calcined sericite | 5 | 0 | 0 |
| Silicic acid anhydride | 0 | 0 | 5 |
| Others | | | |
| Concentrated glycerin | 1 | 1 | 1 |
| | | | |
| Bulk:propellant (LPG) | 5:5 | 5:5 | 5:5 |
| | | | |
| Ester oil (including liquid UV absorber)/solid absorber | 9 | 9 | 9 |
| Ethyl alcohol/ester oil (including liquid UV absorber) | 2.04 | 2.22 | 2.04 |
| Evaluation | | | |
| Powder redispersibility in bulk state | A | A | A |
| Powder redispersibility in aerosol charging | B | A | A |
| Fluffy styling effect (volumized hair) | A | B | A+ |
| Product stability (2 months later) | A | A | A |

From the results shown in Table 4, it is understandable that silicic acid anhydride also can be suitably used as a hair styling powder.

The inventors further studied the behavior of the ultraviolet absorbers in the aerosol cosmetics. Table 5 shows the results.

**[Table 5]**

| Experimental Example | 5-1 | 5-2 | 5-3 | 5-4 | 5-5 | 5-6 | 5-7 | 5-8 |
|---|---|---|---|---|---|---|---|---|
| Ethyl alcohol | 58 | 61 | 62 | 58 | 58 | 57 | 46 | 46 |
| Active agent | | | | | | | | |
| Bisbutyldimethicone polyglyceryl-3 | 3 | 3 | 3 | 3 | 3 | 3 | 3 | 3 |
| Oils | | | | | | | | |
| Pentaerythritol tetra 2-ethylhexanoate | 7 | 4 | 3 | 7 | 7 | 7 | 7 | 7 |
| Diisopropyl sebacate | 7 | 7 | 7 | 7 | 7 | 7 | 7 | 7 |
| UV absorbers | | | | | | | | |
| 2-Ethylhexyl p-methoxycinnamate | 10 | 10 | 10 | 10 | 10 | 10 | 10 | 10 |
| Octocrylene | 1 | 1 | 1 | 1 | 1 | 1 | 1 | 1 |
| Polysilicone-15 | 0 | 0 | 0 | 0 | 0 | 0 | 8.5 | 10 |
| Bis-ethylhexyloxyphenol methoxyphenyl triazine | 1 | 1 | 1 | 1 | 1 | 1 | 4.5 | 3 |
| Diethylamino hydroxybenzoyl hexyl benzoate | 3 | 3 | 3 | 3 | 3 | 4 | 3 | 3 |
| Usability improving powder | | | | | | | | |
| (Vinyl dimethicone/methicone silsesquioxane) crosspolymer | 5 | 5 | 5 | 5 | 5 | 5 | 5 | 5 |
| Hair styling powder | | | | | | | | |
| Talc | 1 | 1 | 1 | 1 | 1 | 1 | 1 | 1 |
| Silicic acid anhydride | 3 | 3 | 3 | 3 | 3 | 3 | 3 | 3 |
| Others | | | | | | | | |
| Concentrated glycerin | 1 | 1 | 1 | 1 | 1 | 1 | 1 | 1 |
| | | | | | | | | |
| Bulk:propellant (LPG) | 5:5 | 5:5 | 5:5 | 5:5 | 5:5 | 5:5 | 5:5 | 5:5 |
| | | | | | | | | |
| Ester oil (including liquid UV absorber)/solid absorber | 625 | 5.50 | 525 | 625 | 625 | 5.00 | 4.47 | 625 |
| Ethyl alcohol/ester oil (including liquid UV absorber) | 2.32 | 2.77 | 2.95 | 2.32 | 2.32 | 2.28 | 1.37 | 1.31 |
| Evaluation | | | | | | | | |
| Bulk stability (2 weeks later) | B | B | B | B | B | B | B | B |
| 0°CProduct stability (2 weeks later) | A | A | A | A | A | A | B | B |
| -5°CProduct stability (2 weeks later) | A | A | A | A | A | A | B | B |

As is obvious from Table 5, in the case of using ethyl alcohol as the main dispersion medium of the bulk, the ester oil in which the solid ultraviolet absorbers are dissolved cause precipitation in the bulk when the amount thereof is 15 times or less the amount of the solid ultraviolet absorbers, but do not cause precipitation in aerosol until the amount reaches about 4.5 times (although there is no practical problem in Experimental Example 6-7 below, there is a problem in stability in Experimental Example 5-8).

Therefore, in order to prevent the stickiness, the amount of the ester oil is preferably 30% by mass or less in the bulk (while the amount of the ester ultraviolet absorbers is 15% by mass or less in the bulk), and in order to prevent the precipitation of the solid ultraviolet absorbers in the aerosol, the ester oil is preferably 4.5 to 15 times with respect to the solid ultraviolet absorbers.

Table 6 shows other formulation examples of the present invention. All of examples showed excellent feeling of use and high stability.

**[Table 6]**

| Experimental Example | 6-1 | 6-2 | 6-3 | 6-4 | 6-5 | 6-6 | 6-7 |
|---|---|---|---|---|---|---|---|
| Ethyl alcohol | 61 | 51 | 60 | 57 | 55.2 | 55 | 61 |
| Active agent | | | | | | | |
| Bisbutyldimethicone polyglyceryl-3 | 5 | 5 | 0.5 | 3 | 3 | 3 | 3 |
| PEG-10/dimethicone | 5 | 5 | 0.5 | 3 | 3 | 3 | 3 |
| Oils | | | | | | | |
| Pentaerythritol tetra 2-ethylhexanoate | 0 | 7 | 7 | 7 | 10 | 7 | 1 |
| Diisopropyl sebacate | 4 | 7 | 7 | 7 | 9.5 | 7 | 7 |
| UV absorbers | | | | | | | |
| 2-Ethylhexyl p-methoxycinnamate | 10 | 10 | 10 | 10 | 7 | 10 | 10 |
| Octocrylene | 0 | 1 | 1 | 1 | 1 | 1 | 1 |
| Bis-ethylhexyloxyphenol methoxyphenyl triazine | 0 | 1 | 1 | 0.5 | 1 | 1 | 1 |
| Diethylamino hydroxybenzoyl hexyl benzoate | 5 | 3 | 3 | 0.5 | | 1 | 1 |
| t-Butyl methoxydibenzoylmethane | | | | 0.5 | 0.3 | 1 | 1 |
| Ethylhexyl triazone | | | | 0.5 | | 1 | 1 |
| Usability improving powder | | | | | | | |
| (Vinyl dimethicone/methicone silsesquioxane) crosspolymer | 5 | 5 | 5 | 5 | 5 | 5 | 5 |
| Hair styling powder | | | | | | | |
| Talc | 1 | | | | | | 1 |
| Silicic acid anhydride | 3 | | 3 | | 3 | | 3 |
| | | | | | | | |
| Others | | | | | | | |
| Concentrated glycerin | 1 | 1 | 1 | 1 | 1 | 1 | 1 |
| | | | | | | | |
| Bulk:propellant (LPG) | 1:9 | 1:9 | 3:7 | 3:7 | 5:5 | 5:5 | 6:4 |
| | | | | | | | |
| Ester oil (including liquid UV absorber)/solid absorber | 2.80 | 6.25 | 6.25 | 12.50 | 21.15 | 6.25 | 4.75 |
| Ethyl alcohol/ester oil (including liquid UV absorber) | 4.36 | 2.04 | 2.40 | 2.28 | 2.01 | 2.20 | 3.21 |

## Claims

1. An aerosol cosmetic, which is filled up as a bulk solution together with a propellant in a pressure-resistant container, comprising:
ethyl alcohol that is a main dispersion medium;
an organic ultraviolet absorber; and
an ester oil; wherein
said organic ultraviolet absorber comprises an organic ultraviolet absorber that is solid at room temperature; and
said solid organic ultraviolet absorber is dissolved in said ester oil.

2. The aerosol cosmetic, according to claim 1, wherein
said solid organic ultraviolet absorber comprises bis-ethylhexyloxyphenol methoxyphenyl triazine and/or diethylamino hydroxybenzoyl hexyl benzoate.

3. The aerosol cosmetic, according to claim 1 or 2, wherein
said ester oil is in an amount of 4.5 to 15 times by mass relative to said solid organic ultraviolet absorber.

4. The aerosol cosmetic, according to any one of claims 1 to 3, further comprising:
a non-lipophilic powder for hair styling.

5. The aerosol cosmetic, according to claim 4, wherein
said non-lipophilic powder for hair styling comprises calcined sericite and/or silicic acid anhydride.

6. The aerosol cosmetic, according to claim 4 or 5, wherein
in the case of using said non-lipophilic powder for hair styling, a polyglycerin-alkyl co-modified silicone active agent is used as an active agent in an amount of 1 to 10% by mass in said bulk solution.

7. The aerosol cosmetic, according to claim 1, wherein
said ester oil (including a liquid ester ultraviolet absorber)/the solid ultraviolet absorber ≥ 4.5 (mass ratio) is satisfied; and
said ethyl alcohol/the ester oil (including a liquid ester ultraviolet absorber) ≥ 1.5 (mass ratio) is satisfied.

8. The aerosol cosmetic, according to claim 7, wherein
said polyglycerin-alkyl co-modified silicone active agent is selected from one or more of a group consisting of bisbutyldimethicone polyglyceryl-3, carboxydecyltrisiloxane, and PEG-10/dimethicone.
